# EUROPEAN PATENT SPECIFICATION

(11) **EP 2 811 912 B1**
(45) Date of publication and mention of the grant of the patent: **21.06.2017**
(21) Application number: 13704262.8
(22) Date of filing: 17.01.2013
(51) Int. Cl.: A61B 17/00

(54) **BALLOON LOCATION DEVICE MANIFOLD FOR VASCULAR CLOSURE DEVICE**
VERTEILER EINER BALLONPOSITIONIERUNGSVORRICHTUNG FÜR EINE GEFÄSSVERSCHLUSSVORRICHTUNG
COLLECTEUR DE DISPOSITIF DE POSITIONNEMENT DE BALLONNET POUR UN DISPOSITIF DE FERMETURE VASCULAIRE

(30) Priority: 24.01.2012 US 201261590000 P
(43) Date of publication of application: 17.12.2014
(73) Proprietor: St. Jude Medical Puerto Rico LLC, Cagus 00726 (PR)
(72) Inventor: TEGELS, Zachary J., Minneapolis, MN 55419 (US); VIDLUND, Robert M., Forest Lake, MN 55025 (US)
(74) Representative: Boult Wade Tennant
(86) International application number: PCT/US2013/021806
(87) International publication number: WO 2013/115993

(56) References cited:
- WO-A1-95/33510
- US-A1- 2002 077 595
- US-A1- 2004 249 342
- US-A1- 2007 083 158
- US-A1- 2008 009 794

## Description

### TECHNICAL FIELD

The present disclosure relates generally to systems for sealing tissue punctures, and more particularly, to systems for indicating balloon inflation pressure in a tissue puncture closure device.

### BACKGROUND

Various surgical procedures are routinely carried out intravascularly or intraluminally. For example, in the treatment of vascular disease, such as arteriosclerosis, it is a common practice to access the artery and insert an instrument (*e.g*., a balloon or other type of catheter) to carry out a procedure within the artery. Such procedures usually involve the percutaneous puncture of the artery so that an insertion sheath may be placed in the artery and thereafter instruments (*e.g*., catheters) may pass through the sheath to an operative position within the artery. Intravascular and intraluminal procedures unavoidably present the problem of stopping the bleeding at the percutaneous puncture after the procedure has been completed and after the instruments (and any insertion sheaths used therewith) have been removed. Bleeding from puncture sites, particularly in the case of femoral arterial punctures, is typically stopped by utilizing vascular closure devices.

While there are a variety of prior art devices and techniques for closing such punctures, one method includes temporarily sealing the tissue puncture intravascularly using an inflation balloon. A sealing material may be delivered to an outer surface of the tissue to seal the tissue puncture while the temporary seal from the balloon is maintained. Challenges exist in confirming proper inflation pressure and positioning of the balloon to maintain the temporary seal.

In United States Patent Application Publication US 2004/0249342, there is described an apparatus for sealing a puncture communicating with a blood vessel including an inner member slidable within an outer member, and a balloon coupled to distal ends of the inner and outer members, according to the preamble of claim 1. In United States Patent Application Publication US 2008/0009794, there is described an apparatus for providing hemostasis within a puncture through tissue including an elongate member having a lumen extending between proximal and distal ends thereof, an expandable member carried on the distal end and a housing on the proximal end, the housing including an interior communicating with the lumen, and further including a valve assembly with a one-way valve allowing access into the housing interior upon application of a pressure differential across the valve, and a movable plunger for overriding and opening the valve.

In International Patent Application Number: PCT/US95/07920, there is described a device for preventing over-inflation of a balloon catheter. A movable wall is attached to a spring for defining a pressure release chamber between the movable wall and a pressure release port of the device.

In United States Patent Application Publication US 2002/0077595, there is described a catheter having a balloon for occluding the coronary sinus and a lumen for delivering fluid distal to the occluding member. The catheter has a proximal portion which is relatively stiff to provide column strength to facilitate insertion, advancement and steering of the catheter.

### SUMMARY

One aspect of the present disclosure relates to a vessel puncture closure device that includes a housing, an inflation tube, a balloon member, an indicator member, and a delivery tube. The inflation tube defines an inflation lumen and extends from the housing. The balloon member is positioned at a distal end of the inflation tube and is arranged in fluid communication with the inflation lumen. The indicator member is positioned in and movable relative to the housing in response to a pressure condition in the
balloon member. The delivery tube is connected to the indicator member and balloon member, and extends through the housing and inflation tube.

The delivery tube may be connected to a distal end of the balloon member. The delivery tube may be configured to deliver a sealing material to a location adjacent the balloon member. The housing may include a transparent material to permit visualization of the indicator member. A position of the indicator member within the housing may represent a fluid pressure in the balloon member. The indicator member may include a piston construction.

The vessel puncture closure device may include a delivery manifold positioned on the delivery tube proximal of the housing. The vessel puncture closure device may further include an inflation manifold positioned on the housing distal of the indicator member. The vessel puncture closure device may include a sheath that defines a passage through which the inflation lumen passes to position the balloon member distal of the sheath. The vessel puncture closure device may include a biasing member positioned in the housing and operable to bias the indicator member distally.

Another aspect of the present disclosure relates to a vessel puncture closure device that includes an inflation tube, a balloon member, an indicator member, and a delivery tube. The inflation tube defines an inflation lumen and is coupled to an inflation hub. The balloon member is positioned at a distal end of the inflation tube and is arranged in fluid communication with the inflation lumen. The indicator member is positioned proximal of the inflation tube and is axially movable relative to the inflation tube in response to a pressure condition in the inflation lumen. The delivery tube is connected to the indicator member and extends through the inflation tube.

The delivery tube may be configured to deliver a sealing material. The indicator member may be biased distally. The indicator member may be in fluid communication with the inflation lumen. The vessel puncture closure device may include a housing to which the inflation tube is connected and within which the indicator member is positioned.

Another aspect of the present disclosure, wich is not part of the invention, relates to a method of operating a vessel puncture closure device. The method includes providing a housing, an inflation tube, a balloon member positioned at a distal end of the inflation tube, an indicator positioned in the housing, and a delivery tube connected to the indicator and extending through the housing and inflation tube. The method also includes positioning the balloon member through a vessel puncture, delivering inflation fluid through the inflation lumen to the balloon member, moving the indicator within the housing to represent a change in inflation pressure in the balloon member, and delivering a sealing material through the delivery tube to the vessel puncture to seal the vessel puncture.

The method may include biasing the indicator member distally within the housing, and moving the indicator member proximally as inflation pressure increases in the balloon member. The method may include connecting a distal end portion of the delivery tube to the balloon member. The method may include providing a sheath defining a passage, and inserting the inflation lumen through the passage to position the balloon member distal of the sheath. The housing may include indicia, and moving the indicator member to a position aligned with the indicia may represent an inflation pressure of the balloon member.

The foregoing and other features, utilities, and advantages of the invention will be apparent from the following detailed description of the invention with reference to the accompanying drawings.

### BRIEF DESCRIPTION OF THE DRAWINGS

The accompanying drawings illustrate various embodiments of the present disclosure and are a part of the specification. The illustrated embodiments are merely examples of the present disclosure and do not limit the scope of the invention.
FIG. 1 is a perspective view of an example vascular closure device in accordance with the present disclosure.
FIG. 1A is a cross-sectional view of the vascular closure device of FIG. 1 taken along cross-section indicators 1A-1A.
FIG. 2 is a partially exploded perspective view of the vascular closure device of FIG. 1.
FIG. 3 is a cross-sectional view of the vascular closure device of FIG. 1.
FIG. 4 is an exploded view of a balloon location device of the vascular closure device of FIG. 1.
FIG. 5 is a perspective view of another example vascular closure device in accordance with the present disclosure.
FIG. 5A is cross-sectional view of the vascular closure device of FIG. 5 taken along cross-section indicators 5A-5A.
FIG. 6 is a partially exploded perspective view of the vascular closure device of FIG. 5.
FIGS. 7-10 illustrate use of the vascular closure device of FIG. 1 with a sheath to seal a vessel puncture in accordance with the present disclosure.

Throughout the drawings, identical reference numbers designate similar, but not necessarily identical, elements.

### DETAILED DESCRIPTION

The systems disclosed herein may be used to close or seal percutaneous punctures made through the body tissue of a patient to gain access to a body cavity of a patient. Access through these percutaneous punctures allows a physician to carry out various procedures in or through the body cavity for examination, surgery, treatment and the like. While not meant to be limiting, the systems are illustrated being used to seal percutaneous punctures that provide access to blood vessels in patients for various procedures. It will be appreciated that the systems are applicable to other procedures requiring sealing of a puncture through body tissue into a cavity including, for example, laparoscopic surgery and other microscopic surgery techniques using a relatively small incision.

As used in this specification and the appended claims, the terms "engage" and "engagable" are used broadly to mean interlock, mesh, or contact between two structures or devices. Likewise "disengage" or "disengagable" means to remove or capable of being removed from interlock, mesh, or contact. A "tube" is an elongated device with a passageway. The passageway may be enclosed or open (*e.g*., a trough). A "lumen" refers to any open space or cavity in a bodily organ, especially in a blood vessel. The words "including" and "having," as well as their derivatives, as used in the specification, including the claims, have the same meaning as the word "comprising."

The general structure and function of tissue closure devices used for sealing a tissue puncture in an internal tissue wall accessible through an incision in the skin are well known in the art. Applications of closure devices including those implementing principles described herein include closure of a percutaneous puncture or incision in tissue separating two internal portions of a living body, such as punctures or incisions in blood vessels, ducts or lumens, gall bladders, livers, hearts, etc.

An exemplary embodiment of the present disclosure includes a vascular closure device having a manifold, delivery tube, and balloon location device. The vascular closure device is used with a sheath that provides access through a vessel puncture and into an inner lumen of the vessel. The delivery tube may include a dual lumen construction. One lumen may be used to deliver a bioadhesive sealant to the tissue puncture. The other lumen may be used as an inflation lumen for delivering inflation fluid to an inflatable balloon positioned at a distal end of the balloon location device. The inflation lumen may also be configured for passage of an inner tube of the balloon location device that extends to the balloon. The inner tube may be connected to the balloon and move axially as the balloon is inflated. Movement of the inner tube is detectable at a housing of the balloon location device at a location proximal of the delivery tube and manifold. The balloon location device may be used to help determine a proper inflation pressure of the balloon. The balloon location device may include a visual indicator that represents the inflation pressure, size or shape of the balloon.

The inner tube may also be used to deliver a secondary bioadhesive sealant to the vessel puncture. The secondary bioadhesive sealant may be used to help seal a tract defined in the first bioadhesive sealant upon removal of the delivery tube from the vessel and tissue puncture. A distal end of the inner tube may extend distally of the balloon. A proximal end of the inner tube may extend proximal to the housing of the balloon location device. An inner tube manifold may be mounted to a proximal end of the inner tube to connect the inner tube with a source of secondary bioadhesive sealant.

The housing of the balloon location device may include a transparent portion that permits some visualization of moving components within the housing. In one example, a piston structure is attached to the inner tube and positioned within the housing. A relative axial position of the piston to the housing may indicate a pressure condition of the balloon.

An interior of the housing may be in fluid communication with a source of inflation fluid used to inflate the balloon. As the pressure within the balloon increases during inflation of the balloon, pressure is exerted on the moveable piston positioned within the housing to move the piston. The distal end of the inner tube may be connected directly to a portion of the balloon. As the balloon changes shape during inflation, the inner tube moves axially, which movement is visible within the housing (*e.g*., via movement of the piston) to represent various inflated positions, pressures, or shapes for the balloon.

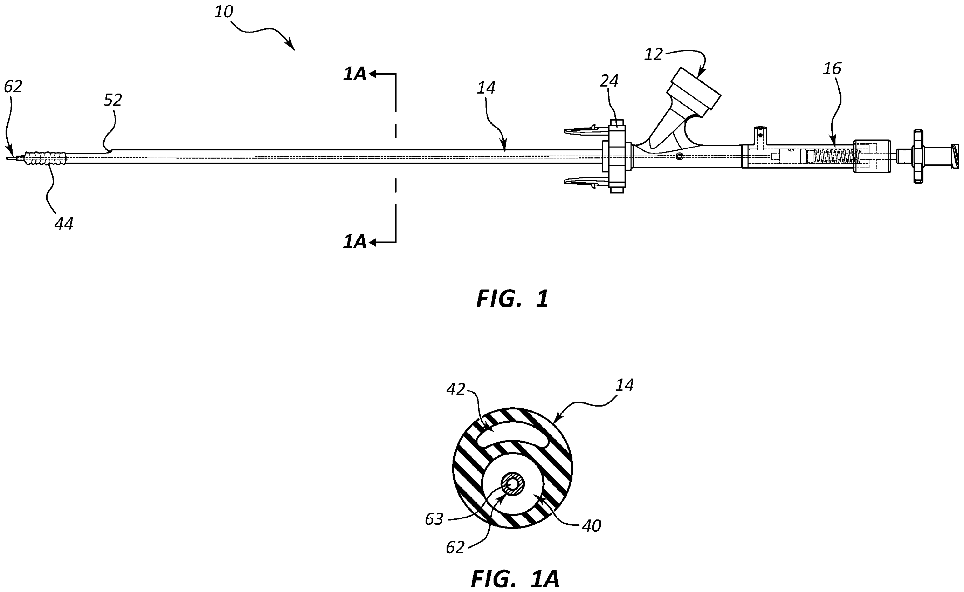

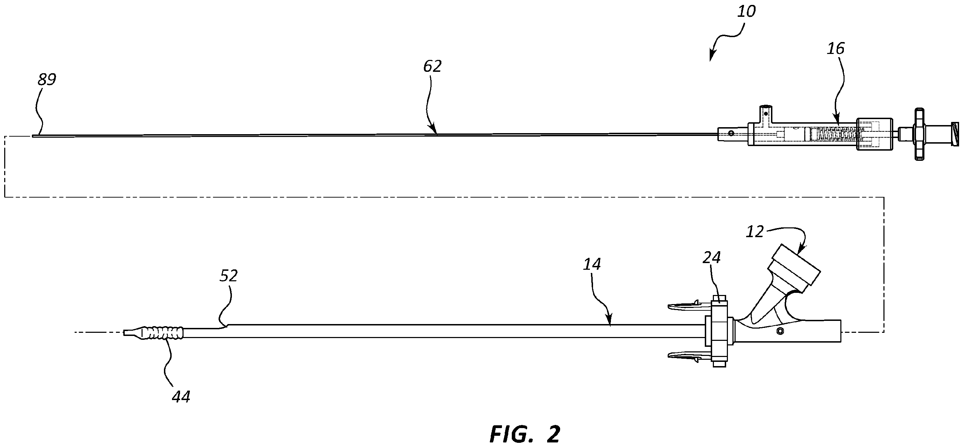

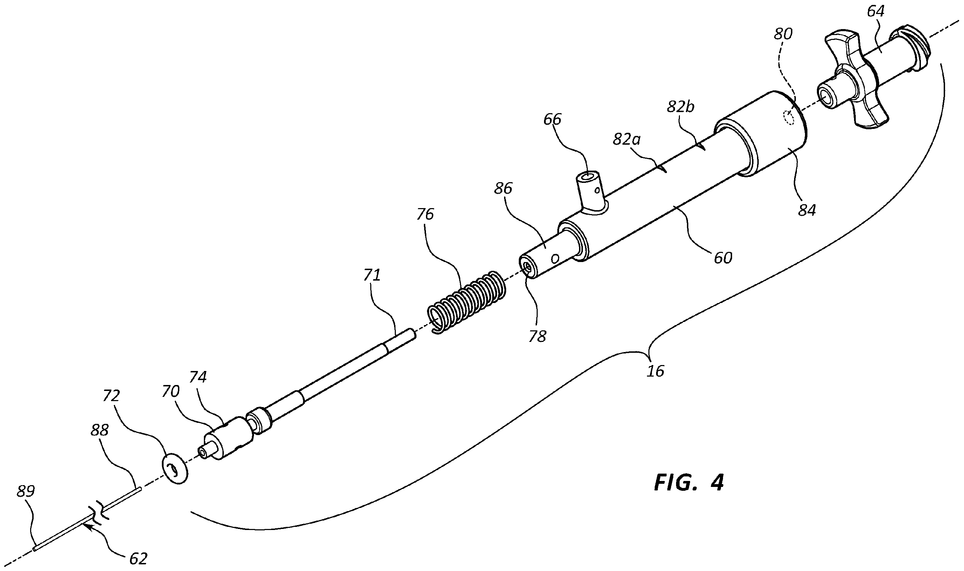

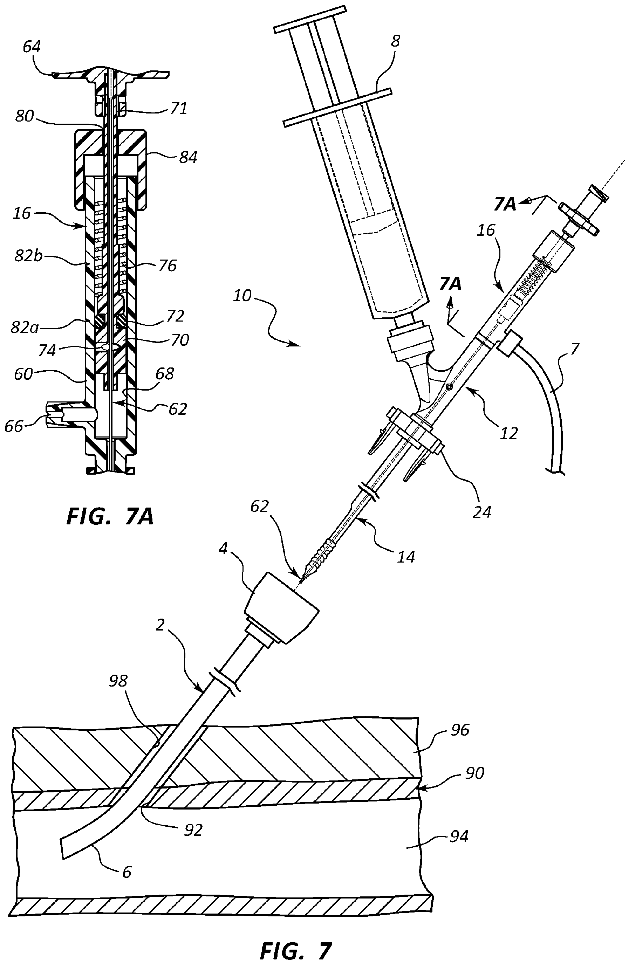

Referring now to FIGS. 1-4, an example vascular closure device 10 is shown and described. The vascular closure device 10 includes a manifold 12, a delivery tube 14, and a balloon location device 16. The vascular closure device 10 may be used with a sheath 2 as shown in FIGS. 7-10 for treatment of a vessel puncture 92 extravascularly. Operation of the vascular closure device may be generally referred to as extravascular closure. The principles disclosed here and related to the vascular closure device 10 may be applicable to other types and methods of closuring punctures in any tissue.

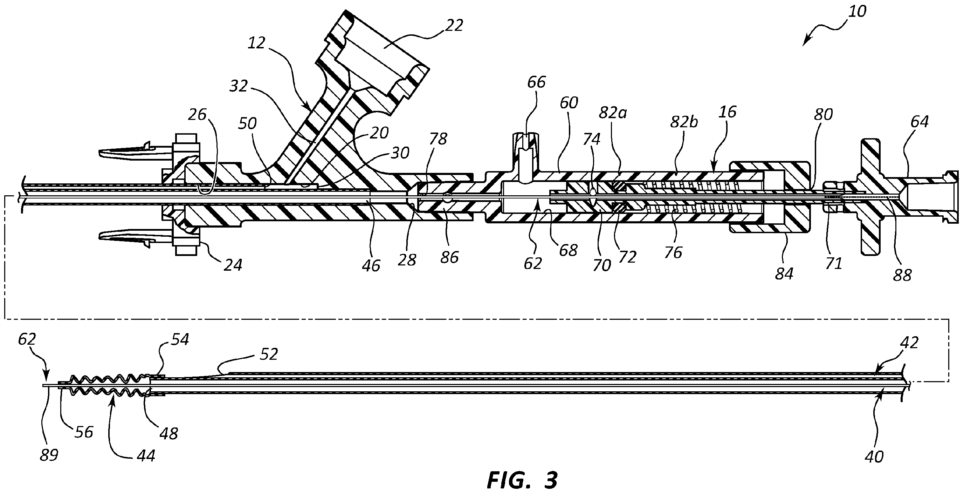

The manifold 12 may include a delivery device passage 20, an injection port 22, and a latch 24 (*see* FIG. 3). The delivery device passage 20 may include a distal opening 26 and a proximal opening or seat 28. The delivery device passage 20 may include a step 30 along its length. The delivery tube 14 may have a stepped construction at its proximal end that mates with the variable sized portions of the delivery device passage 20 defined on opposing sides of the step 30. The delivery tube 14 may be secured to the manifold 12 within the delivery device passage 20. In one example, the delivery tube 14 is connected to the manifold 12 using, for example, an adhesive, sealant, bonding agent, or other device or structure to retain the delivery tube 14 within the delivery device passage. Details concerning a manifold that may be used as manifold 12 are disclosed in U.S. Patent Application No. 61/589,930, filed on 24 January 2012, and entitled "Bioadhesive Delivery Catheter Manifold with Mixing Fixture and Methods".

The distal opening 26 is sized to receive a proximal end of the delivery tube 14. A proximal seat 28 is sized to receive a distal end of the balloon location device 16. The bioadhesive passage 32 of the injection port 22 intersects with the delivery device passage 20. When the delivery tube 14 and balloon location device 16 are mounted to the manifold 12, one of the lumens of the delivery tube 14 is connected in fluid communication with the bioadhesive passage 32 to receive a volume of bioadhesive sealant, and the other lumen of the delivery tube is connected in fluid communication with a source of inflation fluid that is connected to the balloon location device 16.

Latch 24 is configured to releasably attach the vascular closure device 10 to the sheath 2 to limit axial movement of the vascular closure device 10 relative to the sheath 2. In operation, the sheath 2 is first positioned extending through the vessel puncture and into the vessel interior. Inserting the vascular closure device 10 through the sheath 2 and attaching the latch 24 to a hub 4 of the sheath 2 positions a balloon of the vascular closure device distal of a distal end 6 of the sheath 2 and within the vessel interior.

FIG. 3 illustrates the delivery tube 14 having first and second lumens 40, 42 and a balloon 44 positioned at a distal end of the delivery tube 14. The first lumen 40 includes proximal and distal openings 46, 48. The second lumen 42 includes proximal and distal openings 50, 52. Typically, the distal opening of the first lumen 40 is positioned distal of the distal opening 52 of the second lumen 42. The proximal opening 46 of the first lumen 40 may be positioned proximal of the proximal opening 50 of the second lumen 42. As mentioned above, the proximal opening 50 of the second lumen 42 is positioned in fluid communication with the bioadhesive passage 32 of the manifold 12. The distal opening 52 is positioned proximal of the balloon 44 at a location that permits depositing a bioadhesive sealant exterior of the vessel puncture while the balloon 44 is positioned within the vessel and inflated to create a temporary seal with an inner surface of the vessel.

The distal opening 48 of the first lumen 40 is arranged in fluid communication with an interior of the balloon 44. The proximal opening 46 of the first lumen 40 is connected in fluid communication with an interior of a housing of the balloon location device 16 and coupled in fluid communication with a source of inflation fluid.

An inner tube 62 of the balloon location device 16 extends through the first lumen 40 to a location distal of the balloon 44. A proximal waist 54 of the balloon 44 is connected to the delivery tube 14, and a distal waist 56 of the balloon 44 is connected to the inner tube 62 (*see* FIG. 3). Inflating the balloon 44 of the first lumen 40 may cause axial movement of the inner tube 62 as a shape or size of the balloon 44 changes.

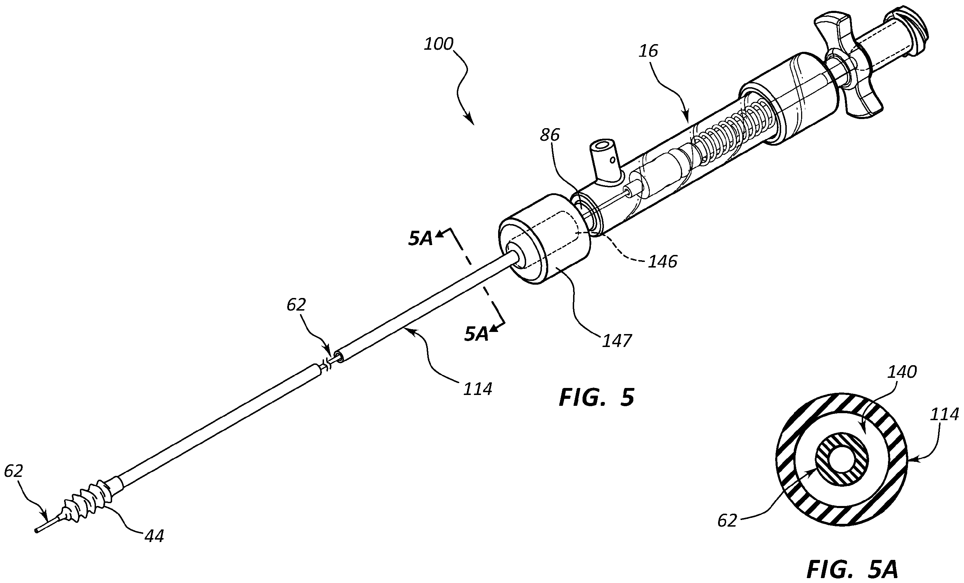

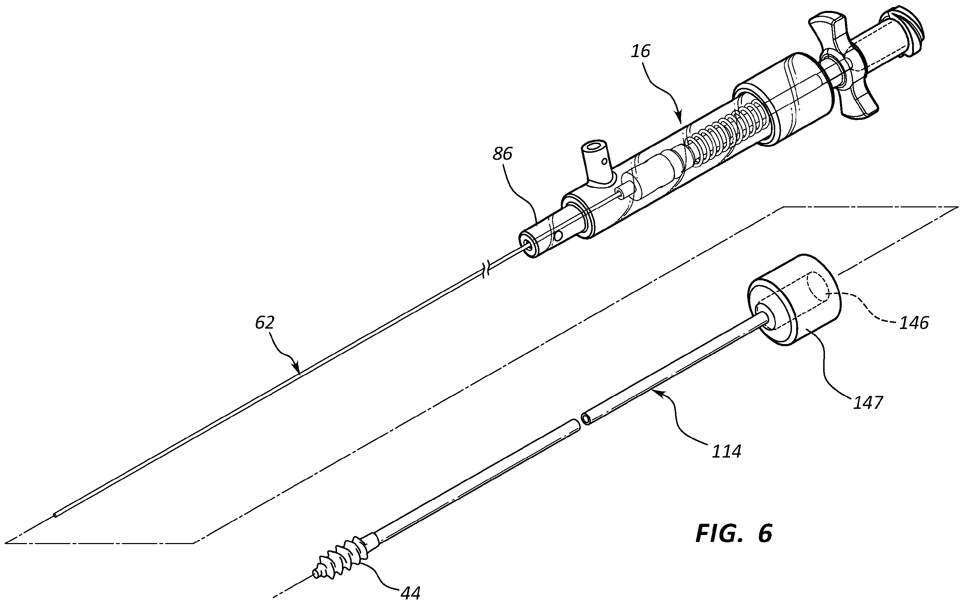

Other delivery tube configurations may be possible in addition to the dual lumen configuration provided in the delivery tube 14 shown in FIGS. 1-4. FIGS. 5-6 illustrate an alternative vascular closure device 100 that includes a delivery tube 114 having a single lumen. The delivery tube 114 includes a lumen 140 having a proximal opening 146 and a hub 147 positioned at a proximal end of the delivery tube 114. The lumen 140 may be configured to deliver a volume of inflation fluid to a balloon 44 positioned at a distal end of the delivery tube 114. The inner tube 62 of the balloon location device 16 may extend through the lumen 140 to a location distal of the balloon 44. A portion of the balloon 44 may be connected directly to the inner tube 62. Another portion of the balloon 44 may be connected directly to the delivery tube 114. A sealant or sealing member may be delivered to the vessel puncture to seal the vessel puncture. A device separate from the delivery tube 114 may be used to deliver the sealant or sealing material. In some arrangements, the delivery tube 114 may include a connection feature such as latch 24 to assist in connecting the vascular closure device 100 to a sheath (e.g., sheath 2) for treatment of a vessel puncture.

The balloon location device 16 may include a housing 60, an inner tube 62, and inner tube manifold 64, an inflation manifold 66 and a piston cavity 68. A piston 70 may be positioned within the piston cavity and extend proximally into contact with the inner tube manifold 64. A seal member 72 and piston connection member 74 may be connected to the piston 70. A biasing member 76 may act on the piston 70 within the piston cavity 68. The housing 60 may include distal and proximal openings 78, 80 that provide access to the piston cavity 68.

The housing 60 may include indicia 82a,b that provide a reference on the housing 60 for determining movement of the piston 70 within the housing 60. The indicia 82a,b may include markings along an exterior surface of the housing. Aligning the piston 70 with various indicia 82a,b may represent different inflation pressures within the balloon 44. The housing 60 may comprise a transparent or translucent material that permits visualization through a wall of the housing 60 to be able to see portions of the piston 70.

The distal and proximal openings 78, 80 may be positioned at proximal and distal ends 84, 86 of the housing 60. The distal end 86 may extend into the proximal seat 28 of the manifold 12. The distal end 86 may have an interference fit with the proximal seat 28. In some arrangements, the distal end 86 is connected within the proximal seat 28 using an adhesive or other bonding agent.

The inner tube 62 may extend completely through the housing 60 with a proximal end 88 of the inner tube 62 extending proximal of the proximal end 84 and out through the proximal opening 80. A distal end 89 of the inner tube 62 may extend distally through the distal opening 78, through the manifold 12, and through the delivery tube 14 to a position distal of the balloon 44 (*see* FIG. 3).

The inner tube 62 may define an inner tube lumen 63 (*see* FIG. 1A). The inner tube lumen 63 may provide a path to deliver, for example, a bioadhesive sealant to the vessel puncture. Alternatively, the inner tube lumen 63 may define a guide wire passage through which a guide wire extends. A suture may extend through the inner tube lumen 63 and be used to operate other features of the vascular closure device 10 at a distal end of the inner tube 62.

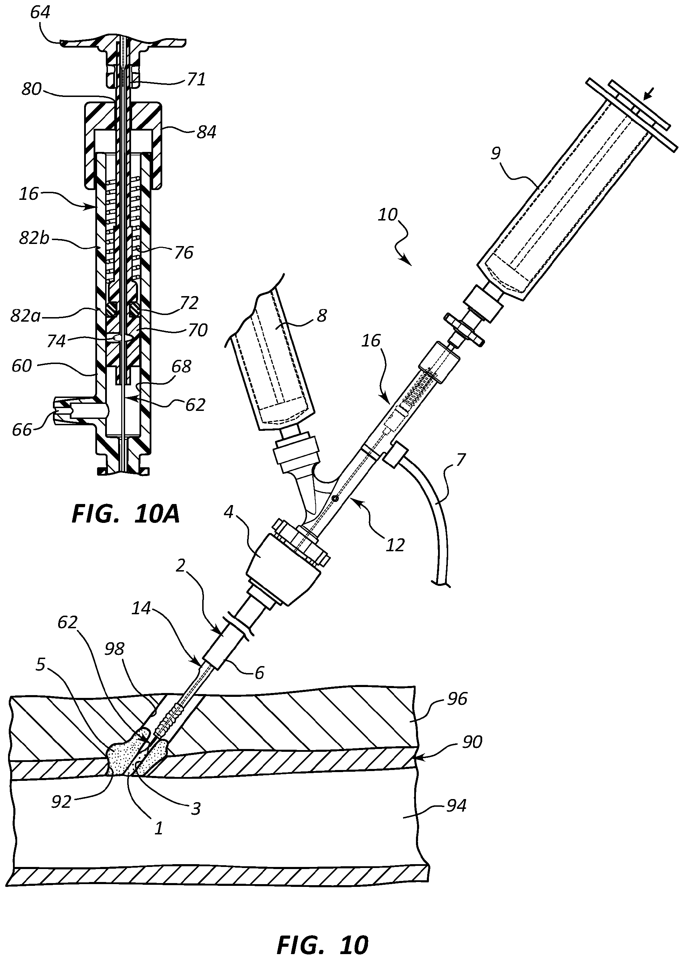

The inner tube manifold 64 may be connected to the proximal end 88 of the inner tube 62 (*see* FIG. 3). The inner tube manifold 64 may include a connection feature such as a luer lock that assists in connecting a device (*e.g.*, a bioadhesive sealant carrier) in fluid communication with the inner tube 62. In one example, a secondary bioadhesive sealant carrier 9 may be connected to the inner tube manifold 64 to deliver a secondary bioadhesive sealant through the inner tube lumen 63 to the vessel puncture (*see* FIG. 10). A proximal end 71 of the piston 70 may also be connected to the inner tube manifold 64. The inner tube 62 is typically connected to the piston 70 at at least one location. Connecting the inner tube manifold 64 directly to the piston 70 may provide concurrent movement of the secondary bioadhesive carrier 9 with the inner tube 62 and piston 70. Connecting the piston 70 to the inner tube manifold 64 may provide an improved connection of the inner tube manifold 64 to the vascular closure device 10.

The piston 70 may be positioned within the piston cavity 68 and move axially therein. The seal member 72 may provide a fluid tight interface between the piston 70 and the walls defining the piston cavity 68. The seal member 72 may limit passage of the inflation fluid, which is positioned at a distal end of the piston cavity 68, from moving rearward past the piston 70 and out through the proximal opening 80 of the housing 60.

The piston connection member 74 may be used to connect the piston 70 to the inner tube 62. The piston connection member 74 may include, for example, a fastener, clamp, adhesive, or other connecting member. The piston 70 may be connected to the inner tube 62 prior to inserting the piston 70 into the piston cavity 68.

The biasing member 76 may bias the piston 70 in a distal or forward direction within the piston cavity 68. Increasing fluid pressure within the balloon 44 may move the piston 70 in a rearward direction against the biasing force of the biasing member 76. In other arrangements, the biasing member 76 may be positioned distal of the piston 70 to bias the piston 70 in a rearward or proximal direction. It may be possible, in some configurations, to provide operation of the balloon location device 16 without the use of a biasing member. In other configurations, separate biasing members are positioned on opposite sides of the piston 70 within the piston cavity 68 to help return the piston 70 to a start or rest position after removal of an inflation pressure (when inflating the balloon 44) or vacuum pressure (when deflating the balloon 44).

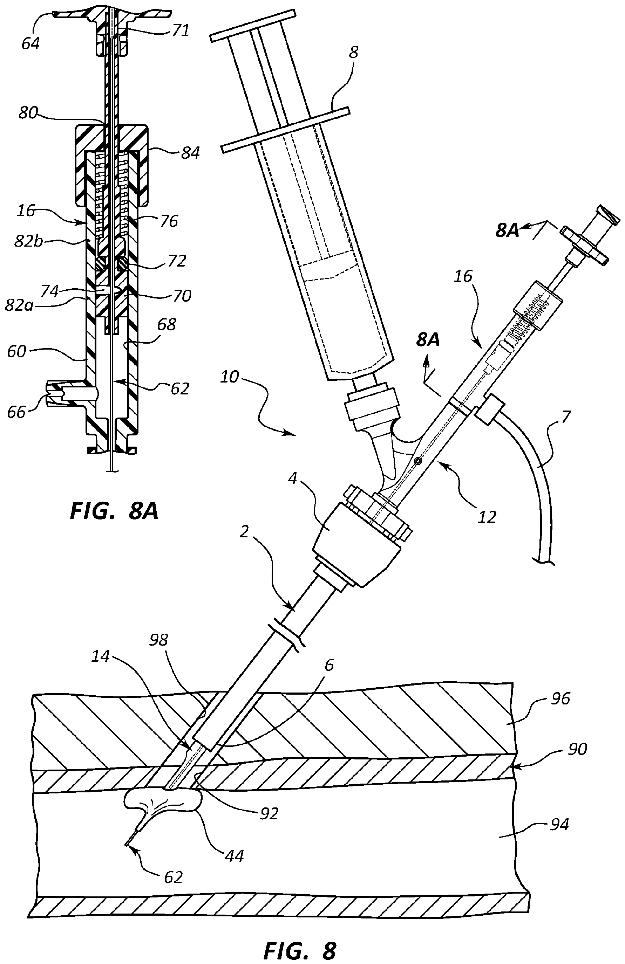

The inflation manifold 66 may be connected to an inflation fluid source 7 as shown in FIGS. 7-10. The inflation fluid source 7 may deliver inflation fluid under pressure through the inflation manifold 66, piston cavity 68, first lumen 40 of the delivery tube 14, and into the balloon 44. The balloon 44 may be deflated by applying a vacuum force to remove the inflation fluid from the balloon 44 through the first lumen 40, piston cavity 68, and inflation manifold 66. The vacuum force may be connected in place of the inflation fluid source 7. FIG. 8 shows the balloon 44 in an inflated position. FIGS. 7 and 10 show the balloon in a deflated position.

Referring now to FIGS. 7-10, an example method of operating the vascular closure device 10 to seal a vessel puncture 92 is shown and described. FIGS. 7-10 illustrate the vascular closure device 10 and sheath 2 extending through a vessel puncture 92 and into a vessel lumen 94 of vessel 90. The vascular closure device 10 and sheath 2 extend through a tissue tract 98 of a tissue layer 96 to access the vessel puncture 92. The tissue tract 98 may be referred to as a percutaneous incision.

In a first operational step, a distal end 6 of the sheath 2 is advanced through the tissue tract 98 and vessel puncture 92 and into the vessel lumen 94. The vascular closure device 10 is aligned with an opening into a hub 4 of the sheath 2 for insertion into the sheath. Prior to inserting the vascular closure device 10 into the sheath 2, the delivery tube 14 is connected to the manifold 12, and the balloon location device 16 is advanced through the manifold 12 and delivery tube 14 and connected to a proximal end of the manifold 12.

Referring to FIG. 8, the delivery tube 14 is advanced through the sheath 2 and the latch 24 is connected to the hub 4 of the sheath 2. The balloon 44 is inflated by delivering a volume of inflation fluid from the inflation fluid source 7, through the housing 60 of the balloon location device 16, through the first lumen 40, and into the balloon 44. The vascular closure device 10 and sheath 2 are retracted (*e.g*., withdrawn proximally) to bring the inflated balloon 44 into contact with an inner surface of the vessel 90 adjacent to the vessel puncture 92. The inflated balloon 44 provides a temporary seal with the vessel 90 to limit blood flow through the vessel puncture 92 from within the vessel lumen 94.

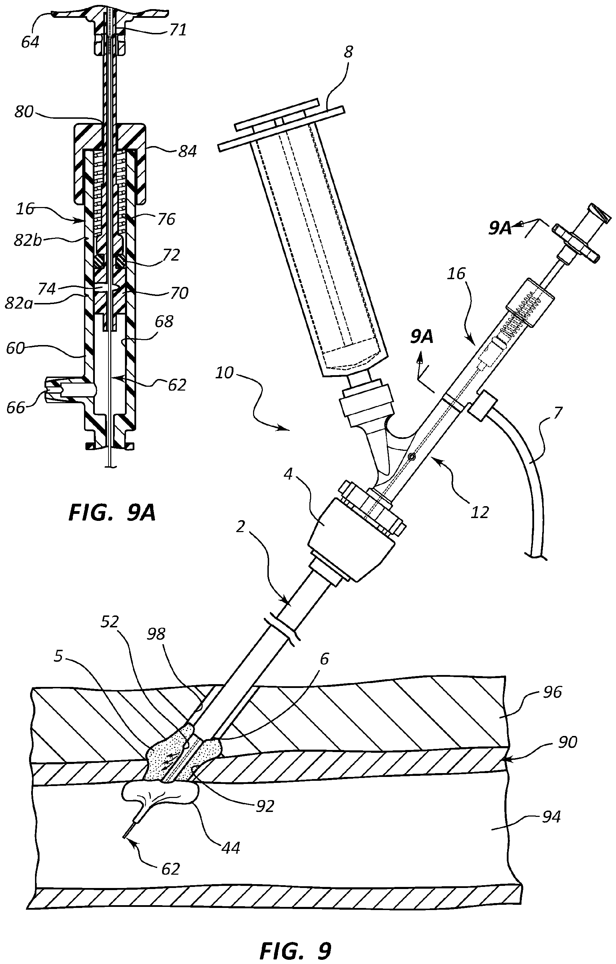

Referring to FIG. 9, a bioadhesive sealant is delivered to the vessel puncture 92 and tissue tract 98. A source of bioadhesive sealant is provided by a first bioadhesive carrier 8 that is connected to the injection port 22 of the manifold 12. Operating the first bioadhesive carrier 8 delivers a volume of the first bioadhesive sealant through the manifold 12 and second lumen 42, and out the distal opening 52. The first bioadhesive sealant forms a bioadhesive plug 5 that seals closed the vessel puncture 92 and tissue tract 98 from outside of the vessel 90. The first bioadhesive material may be allowed to at least partially cure into a solid or semi-solid state that limits movement of the first bioadhesive material into the vessel lumen 94 upon deflating the balloon 44.

Referring to FIG. 10, the balloon 44 is deflated by withdrawing the inflation fluid through the first lumen 40, balloon location device 16, and inflation fluid source 7. The vascular closure device 10 and sheath 2 are further retracted or withdrawn so that the delivery tube 14 is positioned proximal of the bioadhesive plug 5. A tract 3 may be defined within the bioadhesive plug 5 after removal of the delivery tube 14. The tract 3 may be filled by delivering a second bioadhesive sealant via the inner tube 62. A secondary bioadhesive carrier 9 may be connected to the inner tube manifold 64 and operated to deliver a volume of second bioadhesive sealant through the inner tube 62 and into the tract 3. The second bioadhesive sealant may form into a secondary bioadhesive plug 1 within the tract 3 to provide further sealing of the vessel puncture 92.

After delivering of the second bioadhesive plug 1, the entire vascular closure device 10 and sheath 2 are removed from the tissue tract 98 and the sealing procedure is completed.

In some arrangements, a sealing tip or plug is removably attached to the distal end 89 of the inner tube 62. This sealing tip may be disposed or lodged within the tract 3 using a mechanical release device. At least one suture may extend through the inner tube 62 and operate to release the sealing tip within the tract 3. An example detachable sealing tip is disclosed in U.S. Patent Application No. 61/590,027 filed on 24 January 2012 and entitled "Bioresorbable Tip with Low Force Release and Methods".

The balloon location device 16 described herein may provide multiple functions. One function relates to achieving a proper balloon shape at a desired inflation pressure with a visual inspection outside of the patient and independent of the source of inflation fluid. The inner tube of the balloon location device 16 may be connected directly to the balloon (*e.g*., a distal waist of the balloon 44). Inflating the balloon changes a size or shape of the balloon, which moves the inner tube axially. This axial movement is visible within the housing 60.

The balloon location device 16 may also provide an additional lumen for delivering a secondary bioadhesive sealant to the vessel puncture. The inner tube 62 may include this additional lumen as the inner tube lumen 63. The inner tube lumen 63 may be accessible from outside of the housing 60 and at a location proximal of the housing 60. The inner tube manifold 64 may provide connection of a source of secondary bioadhesive sealant to the inner tube lumen 63.

The balloon location device 16 may be configured to permit axial movement of the inner tube and the source of secondary bioadhesive sealant relative to the housing 60. The inner tube 62 may be connected to a piston 70 that is movable within the housing 60 to provide the visual indication of the balloon inflation pressure and the balloon shape. The piston may carry at least one sealing member (*e.g*., O-ring) that limits proximal flow of inflation fluid through the housing 60. The housing 60 may include indicia that represent various balloon inflation pressures or balloon shapes when aligned with portions of the piston 70.

In some arrangements, the inner tube lumen 63 may be used to deliver one or more sutures to a location at a distal end of the vascular closure device 10. In one example, the suture extends through the inner tube lumen 63 to the distal end 89 of the inner tube 62 where the suture is used to release a detachable sealing tip described above. The inner tube lumen 63 may provide several functions such as, for example, providing a pathway for the suture and for delivering a secondary bioadhesive sealant in successive operation steps for the vascular closure device 10.

The bioadhesive materials discussed herein may comprise a single component, or may comprise multiple sealant components that are mixed together. The multiple sealant components may further react together to form a crosslinked network. The sealant components may be naturally derived or synthetic. Some example synthetic components include polyethers such as polyethylene glycol, polypropylene glycol and polytetrahydrofuran. Other examples of synthetic components may include polyamine compositions such as polyvinylpyrrolidones, polyethylene imines and hydrogenated polyacrylonitriles. Other example sealant components include polyacrylic and methacrylic compounds such as polyacrylic acid. Example naturally derived components include protienaceous compositions such as albumin, collagen and polylysine. Other examples include carbohydrate compositions such polyhyaluronic acid. The sealant components may also contain reactive functional groups to promote chemical crosslinking. The sealant components may be cross-linked by any known method including, for example, condensation reactions, Michael addition, and free radical. Functional groups used for cross-linking may include, for example, thiols, acrylates, amines, succinimydyls and aldehydes, to name a few.

The preceding description has been presented only to illustrate and describe exemplary embodiments of the invention. It is not intended to be exhaustive or to limit the invention to any precise form disclosed. Many modifications and variations are possible in light of the above teaching. It is intended that the scope of the invention be defined by the following claims.

## Claims

1. A vessel puncture closure device (10), comprising:
a housing (60);
a delivery tube (14) comprising an inflation lumen (40), the delivery tube extending from the housing;
a balloon member (44) positioned at a distal end of the delivery tube and arranged in fluid communication with the inflation lumen;
an indicator member positioned in and movable relative to the housing in response to a pressure condition in the balloon member; **characterised by** further comprising:
an inner tube (62) connected to the indicator member and balloon member and extending through the indicator member, housing and inflation lumen.

2. The vessel puncture closure device of claim 1, wherein the inner tube is connected to a distal end of the balloon member.

3. The vessel puncture closure device of claim 1, wherein the inner tube is configured to deliver a sealing material to a location adjacent the balloon member.

4. The vessel puncture closure device of claim 1, wherein the housing includes a transparent material to permit visualization of the indicator member.

5. The vessel puncture closure device of claim 1, wherein a position of the indicator member within the housing represents a fluid pressure in the balloon member.

6. The vessel puncture closure device of claim 1, wherein the indicator member comprises a piston construction.

7. The vessel puncture closure device of claim 1, further comprising a delivery manifold positioned on the delivery tube proximal of the housing.

8. The vessel puncture closure device of claim 1, further comprising an inflation manifold positioned on the housing distal of the indicator member.

9. The vessel puncture closure device of claim 1, further comprising a sheath defining a passage through which the inflation lumen passes to position the balloon member distal of the sheath.

10. The vessel puncture closure device of claim 1, further comprising a biasing member positioned in the housing and operable to bias the indicator member distally.

11. The vessel puncture closure device of claim 1,
wherein the indicator member is positioned proximal of the delivery tube and is axially movable relative to the delivery tube in response to a pressure condition in the inflation lumen.

12. The vessel puncture closure device of claim 11, wherein the indicator member is biased distally.

13. The vessel puncture closure device of claim 11, wherein the indicator member is in fluid communication with the inflation lumen.

14. The vessel puncture closure device of claim 11, further comprising a housing to which the inflation tube is connected and within which the indicator member is positioned.

## Patentansprüche

1. Gefäßpunktionsverschlussvorrichtung (10), die Folgendes aufweist:
ein Gehäuse (60);
einen Zufuhrschlauch (14), der ein Inflationslumen (40) aufweist, wobei sich der Zufuhrschlauch von dem Gehäuse erstreckt;
ein Ballonelement (44), das an einem distalen Ende des Zufuhrschlauchs positioniert ist und in Fluidverbindung mit dem Inflationslumen angeordnet ist;
ein Anzeigeelement, das in dem Gehäuse positioniert ist und relativ dazu ansprechend auf eine Druckbedingung in dem Ballonelement beweglich ist; **dadurch gekennzeichnet, dass** sie ferner Folgendes aufweist:
einen inneren Schlauch (62), der mit dem Anzeigeelement und dem Ballonelement verbunden ist und sich durch das Anzeigeelement, das Gehäuse und das Inflationslumen erstreckt.

2. Gefäßpunktionsverschlussvorrichtung nach Anspruch 1, wobei der innere Schlauch mit einem distalen Ende des Ballonelements verbunden ist.

3. Gefäßpunktionsverschlussvorrichtung nach Anspruch 1, wobei der innere Schlauch dazu ausgestaltet ist, ein Abdichtmaterial an eine Stelle angrenzend an das Ballonelement zu leiten.

4. Gefäßpunktionsverschlussvorrichtung nach Anspruch 1, wobei das Gehäuse ein transparentes Material einschließt, um eine Visualisierung des Anzeigeelements zu erlauben.

5. Gefäßpunktionsverschlussvorrichtung nach Anspruch 1, wobei eine Position des Anzeigeelements innerhalb des Gehäuses einen Fluiddruck in dem Ballonelement darstellt.

6. Gefäßpunktionsverschlussvorrichtung nach Anspruch 1, wobei das Anzeigeelement eine Kolbenkonstruktion aufweist.

7. Gefäßpunktionsverschlussvorrichtung nach Anspruch 1, die ferner eine Zufuhrsammelleitung aufweist, die an dem Zufuhrschlauch proximal des Gehäuses positioniert ist.

8. Gefäßpunktionsverschlussvorrichtung nach Anspruch 1, die ferner eine Inflationssammelleitung aufweist, die an dem Gehäuse distal des Anzeigeelements positioniert ist.

9. Gefäßpunktionsverschlussvorrichtung nach Anspruch 1, die ferner einen Schaft aufweist, der einen Durchgang definiert, durch welchen das Inflationslumen verläuft, um das Ballonelement distal des Schafts zu positionieren.

10. Gefäßpunktionsverschlussvorrichtung nach Anspruch 1, die ferner ein Vorspannelement aufweist, das in dem Gehäuse positioniert ist und dazu betriebsbereit ist, das Anzeigeelement distal vorzuspannen.

11. Gefäßpunktionsverschlussvorrichtung nach Anspruch 1,
wobei das Anzeigeelement proximal des Zufuhrschlauchs positioniert ist und relativ zum Zufuhrschlauch ansprechend auf eine Druckbedingung in dem Inflationslumen axial beweglich ist.

12. Gefäßpunktionsverschlussvorrichtung nach Anspruch 11, wobei das Anzeigeelement distal vorgespannt ist.

13. Gefäßpunktionsverschlussvorrichtung nach Anspruch 11, wobei das Anzeigeelement in Fluidverbindung mit dem Inflationslumen steht.

14. Gefäßpunktionsverschlussvorrichtung nach Anspruch 11, die ferner ein Gehäuse aufweist, mit welchem der Inflationsschlauch verbunden ist und innerhalb welchem das Anzeigeelement positioniert ist.

## Revendications

1. Dispositif de fermeture de ponction de vaisseau (17), comprenant :
un boîtier (60) ;
un tube de délivrance (14) comprenant une lumière de gonflage (40), le tube de délivrance s'étendant à partir du boîtier ;
un élément ballonnet (44) positionné au niveau d'une extrémité distale du tube de délivrance et agencé en communication de fluide avec la lumière de gonflage ;
un élément indicateur positionné dans et mobile par rapport au boîtier en réponse à une condition de pression dans l'élément ballonnet, **caractérisé en ce qu'**il comporte en outre :
un tube intérieur (62) connecté à l'élément indicateur et à l'élément ballonnet, et s'étendant à travers l'élément indicateur, le boîtier et la lumière de gonflage.

2. Dispositif de fermeture de ponction de vaisseau selon la revendication 1, dans lequel le tube intérieur est connecté à une extrémité distale de l'élément ballonnet.

3. Dispositif de fermeture de ponction de vaisseau selon la revendication 1, dans lequel le tube intérieur est configuré pour délivrer un matériau de scellement à un emplacement adjacent à l'élément ballonnet.

4. Dispositif de fermeture de ponction de vaisseau selon la revendication 1, dans lequel le boîtier comprend un matériau transparent pour permettre une visualisation de l'élément indicateur.

5. Dispositif de fermeture de ponction de vaisseau selon la revendication 1, dans lequel une position de l'élément indicateur à l'intérieur du boîtier représente une pression de fluide dans l'élément ballonnet.

6. Dispositif de fermeture de ponction de vaisseau selon la revendication 1, dans lequel l'élément indicateur comprend une construction de piston.

7. Dispositif de fermeture de ponction de vaisseau selon la revendication 1, comprenant en outre un collecteur de délivrance positionné sur le tube de délivrance, proximalement par rapport au boîtier.

8. Dispositif de fermeture de ponction de vaisseau selon la revendication 1, comprenant en outre un collecteur de gonflage positionné sur le boîtier, proximalement par rapport à l'élément indicateur.

9. Dispositif de fermeture de ponction de vaisseau selon la revendication 1, comprenant en outre une gaine définissant un passage à travers lequel la lumière de gonflage passe pour positionner l'élément ballonnet, distalement par rapport à la gaine.

10. Dispositif de fermeture de ponction de vaisseau selon la revendication 1, comprenant en outre un élément de rappel positionné dans le boîtier et opérationnel pour rappeler l'élément indicateur distalement.

11. Dispositif de fermeture de ponction de vaisseau selon la revendication un,
dans lequel l'élément indicateur est positionné proximalement par rapport au tube de délivrance et est mobile axialement par rapport au tube de délivrance en réponse à une condition de pression dans la lumière de gonflage.

12. Dispositif de fermeture de ponction de vaisseau selon la revendication 11, dans lequel l'élément indicateur est rappelé distalement.

13. Dispositif de fermeture de ponction de vaisseau selon la revendication 11, dans lequel l'élément indicateur est en communication de fluide avec la lumière de gonflage.

14. Dispositif de fermeture de ponction de vaisseau selon la revendication 11, comprenant en outre un boîtier auquel le tube de gonflage est connecté, et à l'intérieur duquel l'élément indicateur est positionné.
